Europäisches Patentamt

European Patent Office

Office européen des brevets

(11) Publication number : **0 401 922 B1**

# EUROPEAN PATENT SPECIFICATION

(45) Date of publication of patent specification :
16.09.92 Bulletin 92/38

(51) Int. Cl.⁵ : **C10G 32/00, C12P 39/00, C12P 3/00**

(21) Application number : **90201415.8**

(22) Date of filing : **05.06.90**

(54) **Anaerobic desulphurization process for crude oil and petroleum products.**

(30) Priority : **08.06.89 IT 2081689**

(43) Date of publication of application :
**12.12.90 Bulletin 90/50**

(45) Publication of the grant of the patent :
**16.09.92 Bulletin 92/38**

(84) Designated Contracting States :
**AT BE CH DE DK ES FR GB GR LI LU NL SE**

(56) References cited :
**DD-A- 138 780
US-A- 2 641 564
ZENTRALBL. MIKROBIOL., vol. 141, 1986,
pages 291-300, VEB Gustau Fischer Verlag
Jena; V. ECKART et al.:
"MikrobielleEntschwefelung von Erdöl und
schweren Erdölfraktionen 5. Mitteilung:
Anaerobe Entschwefelung von
Romaschkino-Erdöl"**

(73) Proprietor : **AGIP PETROLI S.p.A.
Via Laurentina 449
I-00142 Roma (IT)**
Proprietor : **Enichem Anic S.r.l.
Via Ruggero Settimo 55
I-90139 Palermo (IT)**

(72) Inventor : **Pifferi, Pier Giorgio
Via Ortolani 3
I-40139 Bologna (IT)**
Inventor : **Lanzarini, Gaetano
Via G. Albini 19
I-40137 Bologna (IT)**
Inventor : **Matteuzzi, Diego
Via Baracca 5
I-40033 Casalecchio Di Reno, Bologna (IT)**
Inventor : **Zecchin, Alessandro
Via Byron 4
I-40128 Bologna (IT)**
Inventor : **Leoncini, Sergio
Via Europa 12
I-20097 San Donato Milanese, Milan (IT)**

(74) Representative : **Roggero, Sergio et al
Ing. Barzanò & Zanardo Milano S.p.A. Via
Borgonuovo 10
I-20121 Milano (IT)**

EP 0 401 922 B1

## Description

This invention relates to a microbic desulphurization process for crude oil and petroleum products.

In particular the present invention firstly provides a new mixed culture of obligative and facultative anaerobic microorganisms isolated from industrial effluents, which is particularly effective in the anaerobic organic sulphur desulphurization of crude oil, petroleum fractions and petroleum products.

The most recent data on atmospheric pollution caused by the emission of sulphur dioxide generated by conventional fuels have further emphasized the stringent need to remove completely or as much as possible the sulphur contained in conventional fuels before their use.

In this respect, crude oil, petroleum fractions and some petroleum products often contain considerable quantities of sulphur (for example crude oil contains a sulphur percentage generally varying between 0.025 and 5%, and this can reach as much as 10% in the heavy fractions).

In addition to elementary sulphur and hydrogen sulphide, nearly 200 different sulphur-containing compounds have been identified, these being substantially in the form of thiocycloalkanes and polythioaromatic compounds with more or less condensed structures of which a constituent unit is benzothiophene, thionaphthene and in some cases polysulphides.

Although low-boiling sulphur compounds such as aliphatic sulphides can be easily removed by simple chemical treatment, the removal of sulphur-containing aromatic compounds represents a serious problem.

The currently used industrial crude oil desulphurization processes do not provide an acceptable solution to this problem. In this respect, the desulphurization of crude oil and/or its residual fractions containing high-boiling sulphur compounds by hydrogenation or other chemical paths requires either very special catalysts or extremely drastic temperature and/or pressure conditions, so that such a process does not find wide application because of its very high cost. In addition the high heavy metal concentration in crude oil limits the use of the hydrodesulphurization process because heavy metals are known to poison the catalysts used in this process.

As the concentration both of sulphur compounds and heavy metals increases in the residues obtained during refining, the problem of removing organic sulphur from residual fuel oil is therefore even more difficult to solve. This explains why in the last fifty years considerable study has been given to the possibility of using microorganisms for the pre-combustion removal of sulphur compounds present in fuels.

Both the use of oxidizing aerobic microorganisms which lead to the formation of water-soluble inorganic forms such as sulphate and sulphuric acid and the use of sulphate reducing anaerobic microorganisms which produce $H_2S$ have been well described in the literature.

In the case of aerobic microorganisms, the production of water-soluble substances in a quantity which in obtaining high desulphurization can reach as much as 70% of the starting material creates great problems with regard to the recovery and upgrading of the products.

In the case of anaerobic microorganisms, the processes which have been described up to the present time (such as USA patent 2,641,564 and German Democratic Republic patent 138,780) require the use of special reactors (fermenters comprising two chambers separated by dividing walls or membranes, which cannot be used for crude oil or certain petroleum fractions) or reaction conditions which are very particular and which can result in further difficulties, such as the use of large quantities of surfactants, the purpose of which is to keep the culture medium and petroleum product in intimate contact but which besides considerably increasing the overall cost of the process create difficulties in the subsequent phase separation.

In addition, as reported in DDR patent 138,780 and by V. Eckart et al. in Zentralbl. Mikrobiol. 141 (1986) pp 291-300, the desulphurization yields obtained using the most active of the mixed cultures isolated by the authors are not satisfactory (the highest desulphurization yield observed on a crude oil with an original sulphur content of 1.8% is 38%, and in general maximum desulphurization yields of 65% are described.

Thus, notwithstanding the immense amount of work done, no proposal has proven to be sufficiently economical to allow its industrial scale development, mainly because of the low biocatalytic efficiency of the microorganisms available up to the present time.

The invention, therefore, provides a mixed desulphurizing bacterial culture, deposited with the DMS (Deutsche Sammlung von Mikroorganismen und Zellkulturen GmbH) and thereby identified by the symbol DSM 5307, compulsorily consisting of anaerobic microorganisms of the <u>Desulfovibrio desulfuricans</u> species and at least one sporogenic anaerobic bacterium of the <u>Clostridium</u> species, and at least one facultative anaerobic microorganism of the <u>Coccus</u> species.

This mixed culture, which has been isolated from samples of industrial effluents of refining plants, when in the presence of hydrogen, is capable of considerably reducing the sulphur content of crude oil, petroleum fractions and sulphur-containing petroleum products.

The invention, consequently, concurrently provides a process for desulphurizing a sulphur-containing hydrocarbonaceous substrate selected from crude oil, petroleum fractions and petroleum products, comprising

2

the steps of physically admixing said subtrate with the culture medium N°163 of the DMS having a pH of from 6,0 to 8,0 and containing $Fe^{++}$ ions, inoculating the resultant non-surfactant-aided emulsion or slurry with the mixed desulphurizing bacterial culture defined above, and maintaining the contact of said culture with said substrate at a volume ratio of the subtrate to the culture medium of from 1/99 to 85/15, for a time of from 2 days to 10 days in a non-oxidizing environment, at a temperature of from 20°C to 40°C, under the amospheric pressure, and in the presence of hydrogen.

Samples of the mixed culture N°5307 as defined above have been filed on the 13th April, 1939, with the Deutsche Sammlung von Mikroorganismen und Zellkulturen.

The desired emulsion or suspension can be prepared without surfactants by treating the mixture of the substrate to be desulphurized and the culture broth in an equipment fitted with a mechanical system of high emulsifying efficiency or in mixers without moving parts, such as mixers based on on an ejector system.

This treatment is conveniently conducted at ambient temperature (20°C-40°C) in a non-oxidizing atmosphere and in the presence of hydrogen, as outlined above. In this manner, a microemulsion or microsuspension can be obtained, which is sufficiently stable during the residence time in the fermentor, this time generally varying from 2 to 10 days.

The aqueous culturing medium used is any of those culturing media which are specific for the conventional sulphate-reducing anaerobic bacteria.

The culture medium N°163 of the DSM (Deutsche Sammlung von Mikroorganismen und Zellkulturen GmbH) has the following composition, expressed in grams per litre:

| | |
|---|---|
| $NH_4Cl$ | 1,0 |
| $Na_2SO_4$ | 1,0 |
| $MgSO_4.7H_2O$ | 2,0 |
| $K_2HPO_4$ | 0.5 |
| DL-sodium lactate (70% w/w) | 2.0 |
| $CaCl_2.2H_2O$ | 0.1 |
| yeast extract | 1.0 |
| $FeSO_4.7H_2O$ | 0.5 |

pH between 6.0 and 8.0.

It has also been found that the results of the process in terms of percentage desulphurization are optimized by maintaining the sulphate ion concentration in the culture medium at a constant suitable value, typically between 0.3 and 1.6 g/l. As the results can be optimized by keeping the sulphate ion concentration approximately constant in the case generally of all mixed cultures containing prevalently an obligative anaerobic bacterium of the species <u>Desulfovibrio desulfuricans</u> together with facultative anaerobic accompanying organisms, a second aspect of the present invention is an anaerobic desulphurization process for crude oil, petroleum fractions and petroleum products which uses mixed cultures consisting prevalently of sulphate reducing microorganisms, anaerobic sporigens and obligative anaerobes in the presence of hydrogen at ambient temperature and pressure, characterised in that the sulphate ion concentration is kept between 0.3 and 1.6 g/litre during the process.

It has also been found that the presence of ferrous ions in addition to the sulphate ions (typically by using $FeSO_4$ in the culture medium) gives considerable advantages, in that it produces the formation of a colloidal iron sulphide precipitate in the reaction mixture which, because of the negative charge on this colloid and the positive charge on the microorganisms, results in a more effective distribution of the microbic load in the reaction medium and a more active adsorption surface for the petroleum fraction.

As this is an effect of general applicability, a third aspect of the present invention is an anaerobic desulphurization process for crude oil, petroleum fractions and petroleum products which uses mixed cultures consisting prevalently of sulphate reducing microorganisms, anaerobic sporigens and obligative anaerobes in the presence of hydrogen at ambient temperature and pressure, characterised in that in addition to sulphate ions, the culture medium contains ferrous ions to allow the formation of a colloidal iron sulphide precipitate.

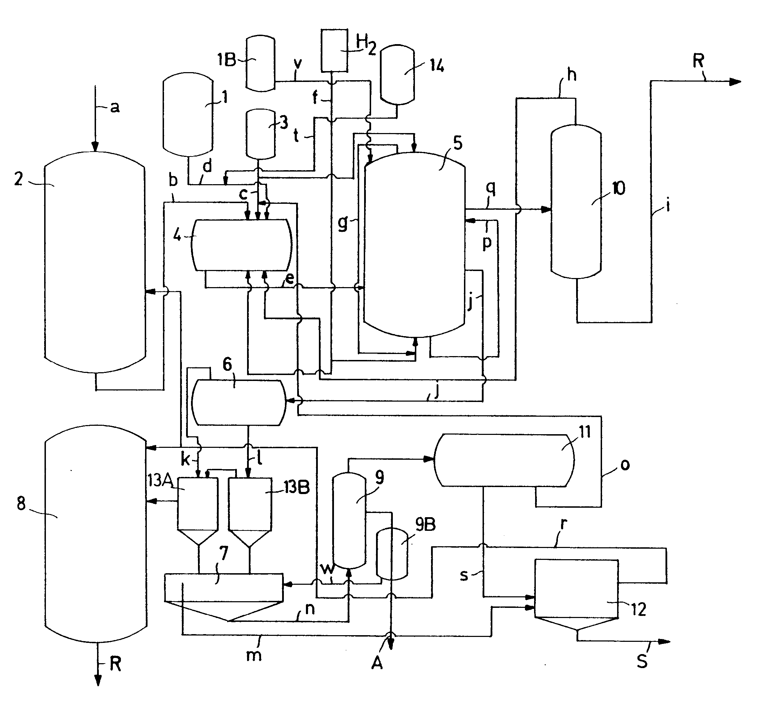

The desulphurization process of the present invention can be conducted on crude oil or on petroleum fractions which have been subjected to thermal treatment in the form of atmospheric or vacuum distillation, including fuel oils, starting from a substrate containing an average sulphur percentage of around 2-3%, to attain a desulphurization percentage of up to 85-90%, or on asphaltenes which have been separated from the petroleum matrix for example by precipitation, or on residues solid at ambient temperature which contain a very high sulphur percentage (of the order of 6-10%), for which high desulphurization percentages (> 20%) are attained. In these latter cases, the process can be performed after predissolving in suitable petroleum fractions (by which the first case then applies) or after predissolving in aprotic polar organic solvents such as tetrahydrofuran, pyridine, dimethylformamide, dimethylacetamide, methylene chloride etc., which on adding the culture broth enable a colloidal asphaltene or residue precipitate to form during the emulsion stage, so obtaining a large contact area between the product to be desulphurized, the culture broth and the microorganisms. The desulphurization process of the present invention can be conducted batchwise or continuously. In this latter case a plant can be used analogous to that shown in the Figure (in this figure all the pump systems necessary for transfer have been omitted). With reference to this figure, in the continuous process the substrate to be desulphurized is fed through line a to the storage tank 2. From here the product is led through line b to the emulsifier 4, to which the following are also fed:

    – through line d, the concentrated culture broth prepared and stored in 1,

    – from the tank 3 and through line c, the concentrated $MgSO_4$ solution to correct the quantity of $SO_4^{--}$ ions,

    – from the tank 14 and through line t, the water for corrective dilution, and

    – through line f, a determined quantity of $H_2$.

The three-phase emulsion (oil/culture broth/$H_2$) is then transferred through line e to the fermentation reactor 5, into which the industrial inoculum stored in 1B is fed through line v. After the established residence time the desulphurized mass passes from the fermentation reactor and through line j to the rapid demulsifier 6.

From this latter, lines k and l carry two streams, one rich in desulphurized product and the other rich in culture broth, which are fed to the sedimentation tanks 13A and 13B. From 13A the oily part passes directly to the storage tank 8 and the aqueous part to the biomass separator 7. From 13B the oily part passes to 13A and the aqueous part to 7. From the separator 7 containing the active recovery biomass, the oily supernatant part is fed to the large-capacity sedimentation tank 12 through line m and the aqueous part (broth + biomass + FeS) is fed to the excess FeS separator 9 through line n. The excess iron sulphide from 9 is washed in 9B, from which the wash liquid is fed through line w to the separator 7 for recovery, and the excess iron sulphide is fed to roasting for production of $H_2SO_4$ and the sulphates required for the process ($MgSO_4$, $FeSO_4$, $Na_2SO_4$) (A), the biomass with the correct FeS content passing to the storage tank 11. From here the biomass is recycled to the emulsifier 4 through line O which is connected into the culture medium feed.

Within the fermentation reactor 5 the $H_2$ and volatile products are recycled through line g and the emulsion is recycled through line p.

The sufficiently desulphurized emulsion is transferred to the demulsifier 6 through line j.

$H_2$ rich in gaseous reaction products ($H_2S$ + volatile products) passes from the top of the fermentation vessel through line q to the separator 10 where the hydrogen is separated from the other products and recycled to the emulsifier 4 as a three-phase gaseous component through line h. The $H_2S$ and the volatile products separated in 10 are then fed to the refinery (R) through line i.

The oily part, collected in the top of the sedimentation vessel 12, is fed through line r to the storage tank 2 or 8 according to the degree of desulphurization attained.

The aqueous part collects in the bottom of the sedimentation tank 12 after this has received the excess biomass from the active biomass storage tank 11, and the entire aqueous fraction which is no longer usable is discharged (S).

The following examples, the only purpose of which is to describe the process of the present invention in greater detail, are in no way to be interpreted as limitative of the scope thereof.

In particular, Examples 1 to 5 relate to tests carried out batchwise on fuel oil samples having different sulphur contents (Examples 1-4) or on separated asphaltenes (Example 5) under different operating conditions.

In Examples 2, 3, 4 and 5, the quantity of $SO_4^{--}$ ions is kept constant by repeatedly adding $MgSO_4$.

Examples 6-13 relate to tests conducted continuously by simulating the aforedescribed bench-scale plant in the laboratory, starting from crude oil (Example 6), fuel oil with different sulphur contents (Examples 7-9) and separated asphaltenes (Examples 10-13).

EXAMPLE 1

The mixed culture DSM 5307 is inoculated into a 2 litre flask containing 1 litre of a mixture of fuel oil and culture broth (having the composition indicated on pages 8 and 9), emulsified under hydrogen and kept under a stream of hydrogen. The conditions under which this test was conducted and the results obtained are summarised in Table 1 below:

## Table 1

| | |
|---|---|
| Temperature | 25°C |
| Oil/culture medium ratio | 2/98 v/v |
| Atmosphere | $H_2$, 1 atm |
| | 80 ml/h |
| pH | 6,5 |
| $SO_4^{--}$ concentration | 0.3 g/l |
| Residence time | 10 days |
| Stirring | 500 rpm |
| Initial sulphur content | 2.63% |
| Desulphurization yield | 48% |
| Production of volatile products | |
| (excluding $H_2S$ and $CO_2$) | 13% |

EXAMPLES 2-4

The general method followed in these examples was substantially the same as in the preceding; however the operating conditions and the results obtained were different, these being shown in Tables 2-4 below.

The initial $SO_4^{--}$ ion concentration is adjusted to the value indicated in each table by increasing the $MgSO_4$ quantity in the culture broth composition given on pages 8 and 9, further $MgSO_4$ quantities being added at the times indicated in order to keep the indicated concentrations unaltered.

EXAMPLE 2

### Table 2

| | |
|---|---|
| Temperature | 25 °C |
| Oil/culture medium ratio | 6/94 v/v |
| Atmosphere | H₂, 1 atm |
| | 120 ml/h |
| pH | 6,9 |
| SO₄⁻⁻ concentration | 0.65 g/l initially |
| | 0.65 g/l after 1st addition after 2 days |
| | 0.65 g/l after 2nd addition after 4 days |
| | 0.65 g/l after 3rd addition after 6 days |
| Residence time | 8 days |
| Stirring | 500 rpm |
| Initial sulphur content | 3.4% |
| Desulphurization yield | 55% |
| Production of volatile products (excluding H₂S and CO₂) | 18% |

EXAMPLE 3

## Table 3

| | |
|---|---|
| Temperature | 35°C |
| Oil/culture medium ratio | 18/82 v/v |
| Atmosphere | H₂, 1 atm |
| | 130 ml/h |
| pH | 7.0 |
| SO₄⁻⁻ concentration | 0.50 g/l initially |
| | 0.50 g/l after 1st addition after 3 days |
| | 0.50 g/l after 2nd addition after 5 days |
| Residence time | 7 days |
| Stirring | 500 rpm |
| Initial sulphur content | 3.0% |

| | |
|---|---|
| Desulphurization yield | 65% |
| Production of volatile products (excluding H₂S and CO₂) | 19% |

EP 0 401 922 B1

## EXAMPLE 4

### Table 4

| | |
|---|---|
| Temperature | 25°C |
| Oil/culture medium ratio | 40/60 v/v |
| Atmosphere | $H_2$, 1 atm |
| | 140 ml/h |
| pH | 7.2 |
| $SO_4^{--}$ concentration | 0.80 g/l initially |
| | 0.80 g/l after 1st addition after 3 days |
| Residence time | 5 days |
| Stirring | 500 rpm |
| Initial sulphur content | 1.5% |
| Desulphurization yield | 70% |
| Production of volatile products (excluding $H_2S$ and $CO_2$) | 21% |

## EXAMPLE 5

A sample of fuel oil (1 kg) is diluted at 35°C with n-hexane (30 l). The asphaltene precipitate obtained, weighing 140 g and containing 6% of sulphur, is separated and dissolved in the minimum quantity of tetrahydrofuran (350 ml). A sample of this solution (100 ml), containing 40 mg of asphaltene, is emulsified with culture broth (600 ml) at pH 7.5, placed in a 250 ml flask and inoculated with the mixed culture.

The parameters used in this procedure and the results obtained are shown in Table 5 below.

8

## Table 5

| Temperature | | 30°C |
|---|---|---|
| Oil/culture medium ratio | | 6.6/93.4 v/v |
| Atmosphere | | $H_2$, 1 atm |
| | | 140 ml/h |
| pH | | 7.5 |
| $SO_4^{--}$ concentration | 0.60 g/l initially | |
| | 0.60 g/l after 1st addition after 3 days | |
| | 0.60 g/l after 2nd addition after 5 days | |
| Residence time | | 6 days |
| Stirring | | 600 rpm |
| Initial sulphur content | | 6.0% |
| Desulphurization yield | | 25% |
| Production of volatile products (excluding $H_2S$ and $CO_2$) | | 15% |

EXAMPLE 6

The plant shown in Figure is simulated, using a reactor of capacity 10 litres. A crude oil sample is used and is fed at a rate of 0.06 l/hour when the plant reaches steady conditions. The test conditions and the results obtained are summarized in Table 6.

## Table 6

| Temperature | 30°C |
|---|---|
| Oil/culture medium ratio | 40/60 v/v |
| Atmosphere | $H_2$, 1 atm |
| | 500 ml/h |

| pH | 7.0 |
| SO$_4^{--}$ concentration | 1.0 g/l constant |
| Residence time | 55 hours |
| Stirring | 300 rpm |
| Initial sulphur content | 0.5% |
| Desulphurization yield | 85% |
| Production of volatile products (excluding H$_2$S and CO$_2$) | 2.8% |

EXAMPLE 7

The method of the preceding example is used, starting from a fuel oil sample which is fed at a rate of 0.11 l/hour when the plant reaches steady conditions.

The test conditions and the results obtained are summarized in Table 7.

### Table 7

| Temperature | 27°C |
| Oil/culture medium ratio | 60/40 v/v |
| Atmosphere | H$_2$, 1 atm |
| | 700 ml/h |
| pH | 7.0 |
| SO$_4^{--}$ concentration | 0.60 g/l constant |
| Residence time | 48 hours |
| Stirring | 300 rpm |
| Initial sulphur content | 3.0% |
| Desulphurization yield | 79% |
| Production of volatile products (excluding H$_2$S and CO$_2$) | 11% |

EXAMPLE 8

The method of Example 6 is used, starting from a fuel oil sample which is fed at a rate of 0.12 l/hour when the plant reaches steady conditions. The test conditions and the results obtained are summarized in Table 8.

Table 8

| Temperature | 25°C |
|---|---|
| Oil/culture medium/H₂ ratio | 83/14.6/2.4 |
| Atmosphere | H₂, 1 atm |
| | 700 ml/h |
| pH | 6.7 |
| SO₄⁻⁻ concentration | 1.20 g/l constant |
| Residence time | 55 hours |
| Stirring | 100 rpm |
| Initial sulphur content | 3.0% |
| Desulphurization yield | 90% |
| Production of volatile products (excluding H₂S and CO₂) | 38% |

EXAMPLE 9

The method of Example 6 is used, conducting the process starting from a fuel oil sample which is fed at a rate of 0.09 l/hour when the plant reaches steady conditions. The test conditions and the results obtained are summarized in Table 9.

Table 9

| Temperature | 32°C |
|---|---|
| Oil/culture medium/H₂ ratio | 65/28/7 |
| Atmosphere | H₂, 1 atm |

11

|                                   | 600 ml/h           |
|-----------------------------------|--------------------|
| pH                                | 7.9                |
| $SO_4^{--}$ concentration         | 1.50 g/l constant  |
| Residence time                    | 60 hours           |
| Stirring                          | 100 rpm            |
| Initial sulphur content           | 2.6%               |
| Desulphurization yield            | 68%                |
| Production of volatile products   |                    |
| (excluding $H_2S$ and $CO_2$)     | 15%                |

EXAMPLES 10-13

A fuel oil sample of high asphaltene content (14% w/w) is diluted with 30 volumes of n-hexane under stirring. The precipitate obtained is dissolved in the ratio of 1:2.5 in the solvents indicated in Table 10. The solutions obtained were subjected to desulphurization by the continuous process of Example 6, with a feed of 7 g/h of asphaltene under steady plant conditions, as follows:

|                                   |                    |
|-----------------------------------|--------------------|
| Temperature                       | 28°C               |
| Oil/culture medium/$H_2$ ratio    | 65/28/7            |
| Atmosphere                        | $H_2$, 1 atm       |
|                                   | 600 ml/h           |
| pH                                | 7.9                |
| $SO_4^{--}$ concentration         | 1.50 g/l constant  |
| Residence time                    | 60 hours           |
| Stirring                          | 100 rpm            |
| Initial sulphur content           | 2.6%               |

The results, in terms of desulphurization yield (%), are also shown in Table 10

## Table 10

| Example No. | 10 | 11 | 12 | 13 |
|---|---|---|---|---|
| Solvent | Tetrahydrofuran | Pyridine | Dimethyl-acetamide | $CH_2Cl_2$ |
| Desulphurization yield | 23.4 | 18.2 | 20.0 | 21.7 |
| Production of volatile products (excluding $H_2S$ and $CO_2$) | 3.5 | 5.0 | 7.5 | 6.3 |

## Claims

1. The mixed desulphurizing bacterial culture, deposited with the DMS (Deutsche Sammlung von Mikroorganismen und Zellkulturen GmbH) and thereby identified by the symbol DMS 5307, compulsorily consisting of anaerobic microorganisms of the Desulfovibrio desulfuricans species and at least one sporogenic anaerobic bacterium of the Clostridium species, and at least one facultative anaerobic microorganism of the Coccus species.

2. Process for desulphurizing a sulphur-containing hydrocarbonaceous substrate selected from crude oil, petroleum fractions and petroleum products comprising the steps of physically admixing said substrate with the culture medium N°163 of the DMS having a pH of from 6,0 to 8,0 and containing $Fe^{++}$ ions, inoculating the resultant non-surfactant-aided emulsion or slurry with the mixed desulphurizing bacterial culture as defined in Claim 1, and maintaining the contact of said culture with said substrate at a volume ratio of the substrate to the culture medium of from 1/99 to 85/15, for a time of from 2 days to 10 days in a non-oxidizing environment, at a temperature of from 20°C and 40°C, under the atmospheric pressure, and in the presence of hydrogen.

3. Process according to Claim 2, wherein the concentration of the $SO_4^{--}$ ion is maintained from 0,3 g/l to 1,6 g/l.

4. Process according to Claim 3, wherein the concentration of the $SO_4^{--}$ ion is kept constant by incremental additions of MgSO4.

5. Process according to Claim 2, wherein the physical admixing step is carried out by stirring.

6. Process according to Claim 2, wherein the physical admixing step is carried out by an ejector-type system.

7. Process according to Claim 2, wherein the contacting step is carried out continuously.

8. Process according to Claim 2, wherein the contacting step is carried out batchwise.

## Patentansprüche

1. Gemischte Entschwefelungs-Backterienkultur, die bei der DMS (Deutsche Sammlung von Mikroorganismen und Zellkulturen GmbH) unter der Identifizierungsnummer DSM 5307 hinterlegt ist und zwingend anaeroben Mikroorganismen der Spezies Desulfovibrio desulfuricans und wenigstens einem sporenbildenden anaeroben Bakterium der Spezies Clostridium sowie gegebenenfalls wenigstens einem anaeroben Mikroorganismus der Spezies Coccus besteht.

**2.** Verfahren zum Entschwefeln eines schwefelhältigen, kohlenwasserstoffhältigen Substrats, ausgewählt unter Rohöl, Erdölfraktionen und Erdölprodukten, umfassend die Stufen des physikalischen Vermischens dieses Substrats mit dem Kulturmedium Nr. 163 von DSM mit einem pH-Wert von 6,0 bis 8,0 und mit einem Gehalt von Fe$^{++}$-Ionen, ein Beimpfen der erhaltenen, nicht mit grenzflächenaktiven Mitteln versetzten Emulsion oder Aufschlämmung mit der gemischten Entschwefelungs-Bakterienkultur, wie in Anspruch 1 definiert, und ein Aufrechterhalten des Kontaktes dieser Kultur mit diesem Substrat bei einem Volumenverhältnis von Substrat zu Kulturmedium von 1/99 bis 85/15 während einer Zeit von 2 Tagen bis 10 Tagen in einer nicht-oxidierenden Umgebung bei einer Temperatur von 20°C bis 40°C unter atmosphärischem Druck und in Anwesenheit von Wasserstoff.

**3.** Verfahren nach Anspruch 2, worin die Konzentration der SO$_4$$^{--}$-Ionen auf 0,3 g /l bis 1,6 g/l gehalten wird.

**4.** Verfahren nach Anspruch 3, worin die Konzentration der SO$_4$$^{--}$-Ionen durch steigende Zugabe von MgSO$_4$ konstant gehalten wird.

**5.** Verfahren nach Anspruch 2, worin das physikalische Zumischen durch Rühren ausgeführt wird.

**6.** Verfahren nach Anspruch 2, worin die physikalische Zumischstufe nach einem Ejektor-Typ-System ausgeführt wird.

**7.** Verfahren nach Anspruch 2, worin die Kontaktierungsstufe kontinuierlich ausgeführt wird.

**8.** Verfahren nach Anspruch 2, worin die Kontaktierungsstufe ansatzweise ausgeführt wird.

**Revendications**

**1.** La culture bactérienne mixte désulfurante, déposée au DSM (Deutsche Sammlung von Mikroorganismen und Zellkulturen GmbH) et désignée par conséquent par le symbole DSM-5307, obligatoirement composée de microorganismes anaérobie de l'espèce <u>Desulfovibrio desulfuricans</u> et d'au moins une bactérie anaérobies sporogène de l'espèce <u>Clostridium</u>, et d'au moins un microorganisme anaérobie facultatif de l'espèce <u>Coccus.</u>

**2.** Procédé de désulfuration d'un substrat hydrocarboné contenant du soufre, choisi parmi une huile brute, des fractions pétrolières et des produits pétroliers, ledit procédé comprenant les étapes consistant à mélanger physiquement ledit substrat avec du milieu de culture N° 163 du DSM, présentant un pH de 6,0 à 8,0 et contenant des ions Fe$^{++}$, à inoculer à la suspension épaisse ou à l'émulsion résultante, non assistée par tensio-actif, la culture bactérienne mixte désulfurante définie dans la revendication 1, et à maintenir ladite culture en contact avec ledit substrat, en un rapport volumique du substrat au milieu de culture valant de 1/99 à 85/15, pendant une durée de 2 jours à 10 jours, dans une atmosphère non oxydante, à une température de 20°C à 40°C, sous la pression atmosphérique et en présence d'hydrogène.

**3.** Procédé conforme à la revendication 2, dans lequel la concentration de l'ion SO$_4$$^{2-}$ est maintenue entre 0,3 g/l et 1,6 g/l.

**4.** Procédé conforme à la revendication 3, dans lequel la concentration de l'ion SO$_4$$^{2-}$ est maintenue constante par addition de MgSO$_4$ par portions.

**5.** Procédé conforme à la revendication 2, dans lequel l'étape de mélange physique est effectuée par agitation.

**6.** Procédé conforme à la revendication 2, dans lequel l'étape de mélange physique est effectuée grâce à un système du type éjecteur.

**7.** Procédé conforme à la revendication 2, dans lequel l'étape de mise en contact est effectuée en continu.

**8.** Procédé conforme à la revendication 2, dans lequel l'étape de mise en contact est effectuée par lots.